(19) Europäisches Patentamt
European Patent Office
Office européen des brevets

(11) Publication number: **0 146 203**
**A2**


(12) # EUROPEAN PATENT APPLICATION


(21) Application number: 84305503.9

(22) Date of filing: 13.08.84

(51) Int. Cl.⁴: **C 07 C 76/02**, C 07 C 79/04

(30) Priority: 19.12.83 US 563208
19.12.83 US 563209
19.12.83 US 563215

(43) Date of publication of application: 26.06.85
Bulletin 85/26

(84) Designated Contracting States: BE CH DE FR GB IT LI LU NL SE



(71) Applicant: W.R. GRACE & CO., Grace Plaza 1114 Avenue of the Americas, New York New York 10036 (US)

(72) Inventor: Wang, Shu-chieh Paul, 6367 Shadowshape Place, Columbia, MD 21045 (US)
Inventor: Sherwin, Martin B., 11500 Karen Drive, Potomac, MD 20854 (US)

(74) Representative: Collier, Jeremy Austin Grey et al, J.A.Kemp & Co. 14, South Square Gray's Inn, London WC1R 5EU (GB)


(54) **Preparation of nitro compounds by vapor phase nitration of oxygenated hydrocarbons.**


(57) Nitro compounds are selectively formed by contacting, at elevated temperature and pressure and in a homogeneous gas phase, an organic oxygenated hydrocarbon having from two to ten carbon atoms with $NO_2$ alone or in the presence of oxygen and/or water.


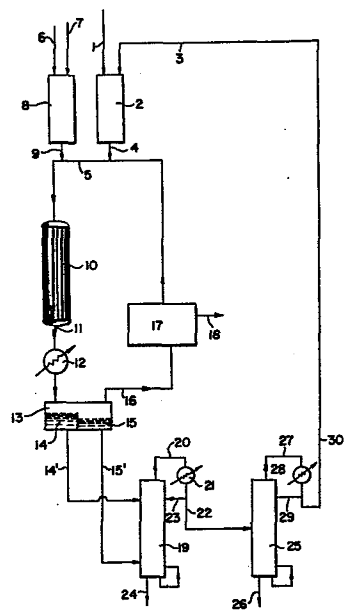



EP 0 146 203 A2

# PREPARATION OF NITRO COMPOUNDS BY VAPOR PHASE NITRATION OF OXYGENATED HYDROCARBONS

The present invention is directed to a process of forming a nitroparaffin and nitroaromatic compounds by gaseous phase reaction of an organic oxygenated hydrocarbon with $NO_2$. The present process provides a method to form pre-selected nitro compounds based on the particular oxygenated hydrocarbon feed. The process further alleviates certain processing steps required in the previously used nitration of hydrocarbon feed such as ethane, propane and the like.

Processes to form nitroparaffins by gaseous phase nitration are known. U.S. Patents 3,780,115 and 3,869,253 teach that nitration of saturated hydrocarbons higher than methane can be accomplished by contacting the hydrocarbon feed with nitrogen dioxide in the presence of oxygen, such as in the form of air. The reactant gases are preheated and then introduced into the reaction zone where the gaseous phase nitration is carried out at elevated pressure and at elevated temperature. The gaseous effluent emitted from the nitration reaction zone is rapidly quenched. The quenched mixture then enters a separator where the gaseous materials in the form of unreacted hydrocarbon, nitric oxide, carbon monoxide and carbon dioxide are removed for subsequent purification and recycling and the remaining phase liquid materials are separated by decantation and the nitroparaffins are recovered by distillation. This nitration process yields a mixture of products having a predominance of nitropropane and nitroethane.

French Publication 78/32,118 discloses that the nitroparaffins product mixture can be made to have an increased yield of nitromethane, the most commercially desired product, by utilizing ethane as the hydrocarbon feed in the homogeneous gas phase nitration. The nitration process can be further enhanced by recycling

into the hydrocarbon feed some of the nitropropane product and/or by conducting the nitration in the presence of an inert gas such as nitrogen, hydrogen or argon.

U.S. Patent 4,260,838, similar to the above French reference, teaches that the gas phase nitration process of U.S. Patents 3,780,115 and 3,869,253 can be improved by altering the feed stock to obtain suitable percentages of different nitroparaffins as suits the needs of the marketplace. This patent teaches that the feed stock be made up of a mixture containing propane, preferably recycled nitroparaffin and possibly inert gas and/or another alkane. The nitrating agent can be either nitrogen dioxide or nitric acid.

Each of the conventional processes, such as those in the above referenced patents, relies on the use of a hydrocarbon feed which provides a nitroparaffin product mixture. These processes have the further defect of providing low yield of nitroparaffin mixture and low selectivity of the most commercially desired compound, nitromethane. Finally, because of the low yield, processes which are based on the gaseous phase nitration of saturated hydrocarbons produce a large volume of gaseous reaction effluents composed predominantly of unreacted hydrocarbon. In order to enhance these prior art processes, the unreacted hydrocarbons must be separated and recovered from the remaining gases, such as by cryogenic means, and then recycled as part of the process feed. Such separation and recovery required additional equipment and adds to the processing costs of the known processes.

A method to selectively form particular nitroalkanes or nitroaromatics from easily available and processable feed is highly desired. It is particularly desired to have a process to selectively form nitromethane, a very industrially useful product.

## Summary of the Invention

An object of the present invention is to provide a process by which a selective nitroparaffin can be formed or that a selective nitro compound is the predominant compound of the resultant products.

Another object of the present invention is to provide a process by which the various unreacted feed materials are readily separated and recyclable.

Another object of the present invention is to provide a process by which one can selectively form nitromethane from readily available and processable materials.

The process of the present invention is capable of selectively forming particular nitrohydrocarbon compounds by contacting in a homogeneous gas phase a $C_2$ to $C_{10}$ oxygenated hydrocarbon with $NO_2$ preferably in the presence of oxygen and/or water.

## Detailed Description of Invention

A process for selectively forming particular aromatic or aliphatic nitro compounds comprises contacting under homogeneous gas phase reaction conditions an oxygenated hydrocarbon with nitrogen dioxide ($NO_2$) preferably in the presence of oxygen and/or water.

The process of homogeneous nitration is generally performed by initially preheating the reactants before they are carried into the reaction zone. The preheating conditions are preferably substantially the same temperature and pressure as the reaction conditions, as fully described below.

The reactant feed of the present process can be selected from an aliphatic or an aromatic or an aliphatic-aromatic oxygenated hydrocarbon compound. The term "oxygenated hydrocarbon" as used in the present

disclosure and in the claims appended hereto refers to compounds having at least one oxygen atom covalently bonded (single or double bond) to a carbon atom, having from two to ten, preferably two to five carbon atoms for aliphatic compounds or seven to ten, preferably seven to eight carbon atoms for aromatic ring containing compounds, having the carbon atom which is bonded to the oxygen be further bonded only to hydrogen and carbon atoms and which are of the class of compounds of alcohols, aldehydes ketones and ethers. The preferred oxygenated hydrocarbon compounds are alcohols, in particular primary alcohols, and aldehydes. In view of nitromethane being the most commercially important product the most preferred compounds have a structure such that at least one carbon atom within the compound which is covalently bonded to an oxygen atom is also bonded to a methyl group such as, for example, acetaldehyde, ethanol, acetone and the like.

The particular structure of the oxygenated hydrocarbon used as the feed in the subject process will be the determinative element as to what nitro compound is formed or what predominant nitro compound is formed from a mixture. For example, when the oxygenated hydrocarbon is selected from an alcohol or an aldehyde represented by

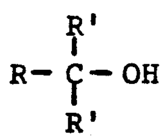

and

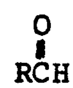

respectively, in which R represents an alkyl, alkaryl or aryl, each R' separately represents R or hydrogen when R is an alkyl or alkaryl and R' represents hydrogen when R is an aryl group, $RNO_2$ will be the sole or dominant product formed. When the oxygenated hydrocarbon is selected from an ether or a ketone represented by

$$R''CH_2-O-CH_2R''' \quad \text{and} \quad R''\overset{O}{\overset{\|}{C}}R'''$$ 

respectively, in which R'' and R''' represents the same alkyl group, one produces $RNO_2$ as either the sole or dominant nitro product. When the pairs R and R' or R'' and R''' of the formulae above represent different hydrocarbon groups, the process will form a mixture solely or predominantly of $RNO_2$ and $R'NO_2$ or $R''NO_2$ and $R'''NO_2$. These mixtures can be separated by distillation. The preferred oxygenated hydrocarbons are above compounds where R and R' are made up of the same hydrocarbon or hydrogen and where R'' and R''' are the same hydrocarbon group. The most preferred oxygenated hydrocarbons are selected from alcohols and aldehydes where R is methyl and R' is hydrogen. When ethers or ketones are used it is preferred that R'' and R''' are each methyl to thereby form nitromethane as the sole product.

Examples of oxygenated hydrocarbons which are useful as a feed in the subject process are alcohols, preferably $C_2$-$C_5$ alcohols, such as ethanol, 1-propanol, 2-propanol, 1-butanol, tert-butanol, 1-pentanol and the like; aldehydes, preferably $C_2$-$C_5$ aldehydes such as acetaldehyde, propionaldehyde, butyraldehyde and the like; ketones preferably $C_3$-$C_5$ ketones such as acetone, diethylketone and the like; and ethers, preferably $C_3$-$C_5$ ether, such as methyl ethyl ether, diethyl ether and the like. The specific compound will be dictated by the desired nitro compound and the economics and availability of the oxygenated hydrocarbon.

The oxygenated hydrocarbon compounds preferably do not contain non-hydrocarbon groups except for the oxygen, as

described above. However, the compounds may contain non-hydrocarbon groups which will not inhibit the subject process, such as nitriles and the like. The supply of oxygenated hydrocarbons may also contain small amounts of other compounds, such as lower or higher homologs of the oxygenated hydrocarbons described above without interferring with the presently obtained unexpected result.

The above described oxygenated hydrocarbon is contacted in the reaction zone with nitrogen dioxide or its precursors, $N_2O_4$ or nitric acid which dissociates to $NO_2$ and water under the reaction zone conditions. These materials are readily obtained commercially.

It is preferred that the feed also includes oxygen, usually in the form of air. The oxygen as well as the nitrogen dioxide can be at least partially obtained from recycled unreacted materials which have been separated and purified by conventional methods from the reaction product as more fully described below.

The feed may further contain inert gas such as nitrogen, carbon monoxide, carbon dioxide, argon or mixtures thereof. Further, the feed can contain water either as part of the carrier for the oxygenated hydrocarbon reactant feed or as a part of the nitrating agent.

The conditions and parameter ranges for conducting the homogeneous gaseous nitration of an oxygenated hydrocarbon are (a) that the reaction zone feed be in a molar ratio of $NO_2$ to oxygenated hydrocarbon of from about 0.3 to 4 or greater and preferably from about 0.5 to 3. The environment can be, therefore, either a reducing or an oxidizing environment depending on the feed ratio used. When oxygen is used as an additional feed, it should be in from about 0.05 to 1 mole per mole of $NO_2$.
The reaction is carried out at elevated temperature of from about 100 to about 500°C and preferably from 150 to 400°C. The reaction is carried out

under elevated pressure of from about 5 to 20 bars with from 5 to 12 bars being preferred. The combined temperature and pressure conditions must be such as to maintain the reactants in a homogeneous gas phase. The inert gases in the feed (A, CO, $CO_2$, $N_2$) can be from about 0 to 90 volume percent. The water can be from about 0 to 30 weight percent based on the $NO_2$ with at most 10 being preferred. The reaction contact time of the reaction gases in the reaction zone can be from about 0.5 to 20 seconds with the order of from about 1 to 12 seconds being preferred.

Referring to the drawing to illustrate the subject process, an oxygenated hydrocarbon such as ethanol, acetaldehyde, etc. is transported from a reservoir (not shown) by pipeline 1 to preheater 2. Preheater 2 is also used to preheat the oxygenated hydrocarbon being recirculated through pipeline 3, as more fully described hereinbelow. The preheater is maintained at substantially the reaction zone entry temperature of about 100 to 500°C and pressure of from about 5 to 20 bars. The preheated oxygenated hydrocarbon is then passed through pipeline 4 to reactor intake pipeline 5. The nitrogen dioxide and the oxygen (as air, when used) are introduced to preheater 8 via pipelines 6 and 7, respectively. The preheater 8 is maintained at temperature and pressure conditions substantially the same as that of preheater 2. The mixed preheated $NO_2/O_2$ gases pass through pipeline 9 to reactor intake pipeline 5 using gas-gas mixing devices such as spargers, venturis, etc. The preheated gases are passed through reactor 10 which may be in the form of a tubular reactor heated by salt at a temperature of from 100° to 500°C, preferably from 150° to 400°C and at a pressure of approximately 1 to 20, preferably about 2 to

12 bars. The reactor effluents withdrawn through pipeline 11 are cooled to ambient temperature in cooler 12 which uses super-cooled water to rapidly cool the gases. The cooled reactor effluents are separated in the separator 13. The liquid effluent separates into organic liquid phase 14 and aqeuous liquid phase 15.

The uncondensed gaseous reaction effluents are removed from the separator 13 through pipeline 16. The uncondensed gaseous reaction effluents obtained in the present process are generally a mixture of components composed predominantly of nitrogen monoxide and inert gases. These effluent gases are distinctly different from those encountered in conventional hydrocarbon gaseous nitration processes where the effluent gases are rich in unreacted hydrocarbons. The hydrocarbons must be separated from the NO (which must be separately treated) and recycled as part of the feed. Such separation is complex and costly. In contrast, the present uncondensed effluent gases of separator 13 are substantially free of unreacted oxygenated hydrocarbon and thereby do not require separation. Instead these gases can be directly treated at station 17 to re-oxidize the nitrogen oxide to nitrogen dioxide for reuse by, for example, directly injecting oxygen into the gaseous effluent. To prevent build-up of inert gases due to the recycling of gaseous effluent, a purge stream 18 is maintained.

The condensed organic and aqueous liquid phases 14 and 15, respectively, are removed from separator 13 and sent by pipelines 14' and 15' to an azeotropic distillation column 19. When the nitro compound product has a lower density than water (i.e. some $C_4$ and higher nitro compounds) the organic and aqueous liquid phases 14 and 15 will be in reversed position in separator 13 to that shown. In such instances (not shown) line 14' will enter

the bottom portion of column 19 and line 15' will enter the top portion of column 19. Azeotropic distillation column 19 normally operates at a pressure of about 1.25 bars or less and at temperatures sufficient to azeotropically distill the nitroalkane or nitroaromatic products as well as other compounds having a boiling point lower than the nitro products, including any unreacted oxygenated hydrocarbon feed with associative water. These materials are passed via pipeline 20, condenser 21 and pipeline 22 to a light oxygenate removal distillation column 25. Some of the distillate may be recycled to the azeotropic column 19 by pipeline 23. The majority of the water and the heavy by-products such as acids and the like are removed as bottom products through pipeline 24. This stream containing heavy by-products may be recycled to reactor 10 via line 3.

The oxygenate removal column 25 operates at a pressure of about 1.25 bars or less and at a temperature range sufficient to remove overhead the oxygenated hydrocarbons such as a temperature range of from 30°C to 95°C. The bottom product of column 25 is removed by pipeline 26 and is composed of a mixture of a major amount of nitro alkanes or mixture of nitroalkanes or nitroaromatic as is appropriate based on the oxygenated hydrocarbon feed used. In addition there may be present a small amount of water (from the prior azeotropic distillation) and trace amounts of oxygenated hydrocarbon by-products. The material removed by pipeline 26 is subsequently chemically treated (not shown) to remove any trace oxygenated contaminants then fed to a dehydration column (not shown) and where a mixture of nitrocompounds are produced to a fractionation column (not shown) to recover pure nitro products. The nitro product of the present process is either composed of a single nitro compound such as

nitromethane or of a mixture of nitro compounds highly selective with respect to one nitro product which is dependent on the starting oxygenated hydrocarbon feed.

The overhead effluent of column 25 is removed by pipeline 27 through condenser 28. Some of the distillate may be recycled to column 25 by pipeline 29. The overhead distillate is made up predominantly of oxygenated hydrocarbons which can be readily recycled via pipeline 30 to preheater 2 as part of the oxygenated hydrocarbon feed.

By utilizing an oxygenated hydrocarbon as the feed material in the subject process it has been unexpectedly found that one can readily form nitroalkane or nitroaromatic compounds, that the nitro compound product will be highly selective based on the particular oxygenated hydrocarbon used as the feed and that the unreacted oxygenated hydrocarbon can be readily separated and recycled as part of the feed to further improve the effectiveness and efficiency of the subject process. The subject process provides a means of custom directing the formation of a preselected nitro compound.

The following examples are given for illustrative purposes only and are not meant to be a limitation on the claims appended hereto. All parts and percentages are by weight unless otherwise indicated.

Example I

A series of production runs were conducted using ethanol as the oxygenated hydrocarbon feed. Each feed material was preheated to 150°C at 10 bars. The nitrogen dioxide and oxygen (when used) were preheated separately from the ethanol and water (when used). The preheated feed materials were then mixed and reacted in a tubular reactor for a residence time of 8 seconds. The reactor effluent was quenched. The nitric oxide, carbon monoxide

and carbon dioxide were removed and the nitric oxide treated with oxygen to obtain nitrogen dioxide which was recycled to the reactor. The remaining liquid was distilled to azeotropically remove the nitro compound and low boiling oxygenated hydrocarbon including unreacted ethanol. The azeotropic distillate was further distilled to separate the nitro compound from the oxygenates. The nitro compound was nitromethane. No other nitro compound was detected.

Table I below lists the reaction condition, feeds and products. The yield of nitromethane is based on moles of nitro compound per total moles of non-recyclable products.

TABLE I

Nitration of Ethanol

| Run No. | 1 | 2 | 3 | 4 |
|---|---|---|---|---|
| **Conditions** | | | | |
| Temperature (°C) | 300 | 400 | 300 | 300 |
| Pressure (atm) | 10 | 10 | 10 | 10 |
| **Feed** (mmoles/hr) | | | | |
| Ethanol | 3860 | 4020 | 4470 | 3860 |
| Nitrogen Dioxide | 940 | 940 | 1190 | 1180 |
| Water | 0 | 0 | 1930 | 1640 |
| Oxygen | 0 | 0 | 0 | 440 |
| Nitrogen | 4680 | 4500 | 3600 | 3130 |
| $NO_2/C_2H_5OH$ | 0.24 | 0.23 | 0.26 | 0.3 |
| **Products** (mmoles/hr) | | | | |
| Nitromethane | 71 | 99 | 61 | 219 |
| Carbon Monoxide | 23 | 82 | 31 | 92 |
| Carbon Dioxide | 72 | 117 | 103 | 245 |
| Ethyl Formate | 13 | 40 | 34 | 107 |
| Acetal | 22 | 41 | 15 | 51 |
| Ethyl Acetate | 5 | 6 | 4 | 58 |
| Acetaldehyde | 456 | 373 | 537 | 1099 |
| Methanol | 87 | 118 | -- | -- |
| Nitric Oxide | 810 | 1413 | 1185 | 963 |
| Oxygen | 0 | 0 | 0 | 28 |
| **Nitromethane** | | | | |
| Yield, %* | 28 | 24 | 31 | 39 |

*Moles of nitromethane/total moles of $C_1$ compounds produced.

## Example II

A series of production runs were conducted in the same manner as in Example I above except that other oxygenated hydrocarbons, acetone and acetaldehyde were used. The reaction conditions, feeds and products are listed in Table II below. The yield was based on a per pass run.

### TABLE II

| Run No | 1 | 2 | 3 | 4 |
|---|---|---|---|---|
| Temperature (°C) | 300 | 350 | 200 | 300 |
| Pressure (atm) | 10 | 10 | 10 | 10 |
| Feed (mmoles/hr) | | | | |
| Acetone | 9579 | 4216 | 0 | 0 |
| Acetaldehyde | 0 | 0 | 4751 | 3734 |
| Nitrogen Dioxide | 1395 | 2275 | 619 | 1747 |
| Water | 1699 | 0 | 0 | 0 |
| Oxygen | 344 | 361 | 352 | 348 |
| Nitrogen | 1948 | 3907 | 3782 | 4255 |
| Moles $NO_2$/Oxy.Cmpd. | 0.14 | 0.53 | 0.13 | 0.47 |
| Product (mmoles/hr) | | | | |
| Nitromethane | 76 | 112 | 200 | 181 |
| Carbon Monoxide | 123 | 400 | 10 | 253 |
| Carbon Dioxide | 376 | 733 | 506 | 476 |
| Methyl Nitrate | 0 | 0 | 13 | 26 |
| Methanol | 26 | 66 | 0 | 0 |
| Acetic Acid | 865 | 816 | 365 | 661 |
| Oxygenated HC | 62 | 30 | 21 | 12 |
| Nitromethane Yield %* | 13 | 9 | 27 | 19 |

*Moles of nitromethane/total moles of $C_1$ compounds produced.

## Example III

A series of production runs were conducted in the same manner as in Example I above except that the oxygenate feed was acetaldehyde and the reaction conditions were varied. Details of the runs are given in Table III below.

### TABLE III

| Run No. | 1 | 2 | 3 | 4 |
|---|---|---|---|---|
| Temperature (°C) | 150 | 190 | 190 | 250 |
| Pressure (atm) | 7.8 | 10.5 | 7.8 | 10.2 |
| Feed (mmoles/hr) | | | | |
| Acetaldehyde | 2020 | 2263 | 2240 | 2265 |
| Nitrogen Dioxide | 1032 | 1198 | 1486 | 1311 |
| Water | 1925 | 1889 | 1886 | 1759 |
| Oxygen | 433 | 303 | 303 | 303 |
| Nitrogen | 4685 | 6587 | 6587 | 6587 |
| $NO_2/CH_3CHO$ | 0.51 | 0.53 | 0.66 | 0.57 |
| Acetaldehyde Conversion (%) | 23.8 | 27.5 | 28.4 | 40.5 |
| Nitromethane Yield, %* | 21.0 | 25.5 | 24.6 | 23.0 |

*moles of nitromethane/total moles of $C_1$ compounds produced

CLAIMS

1. A process for forming nitroalkanes and nitroaromatics which comprises contacting a $C_2-C_{10}$ alcohol, aldehyde, ketone or ether with nitrogen dioxide in a homogeneous gas phase reaction zone at an elevated pressure and at a temperature of from $100^oC$ to $500^oC$, and recovering the formed nitro compounds.

2. A process according to claim 1 wherein an alcohol or aldehyde is used having the formula:

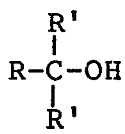 or 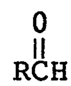

wherein R represents an alkyl, alkaryl or aryl group, each R' separately represents hydrogen or R when R is an alkyl, and R' separately represents hydrogen when R is aryl.

3. A process according to claim 1 wherein a ketone or ether is used having the formula:

$R''CH_2-O-CH_2R'''$ or 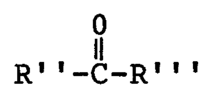

wherein R'' and R''' each represent the same alkyl group.

4. A process according to claim 1, 2 or 3 wherein an alcohol, aldehyde, ketone or ether is used having 2 to 5 carbon atoms.

5. A process according to claim 1 wherein ethanol, acetone or acetaldehyde is contacted with nitrogen dioxide.

6. A process according to any one of claims 1 to 5 wherein the reaction zone further contains oxygen, water or both.

7. A process according to any one of claims 1 to 6 wherein the reaction zone effluent is cooled, the resulting liquid phase effluent is separated from the non-condensed gaseous effluent, and any alcohol, aldehyde, ether or ketone is recovered and returned at least in part to the reaction zone.

8. A process according to any one of claims 1 to 7 wherein the reaction zone pressure is from about 5 to 12 bars, the temperature is from about 180-400$^{o}$C, the molar ratio of oxygen to nitrogen dioxide is from about 0.05 to 1 and the molar ratio of nitrogen dioxide to alcohol, aldehyde, ether or ketone is from about 0.3 to 3.

0146203

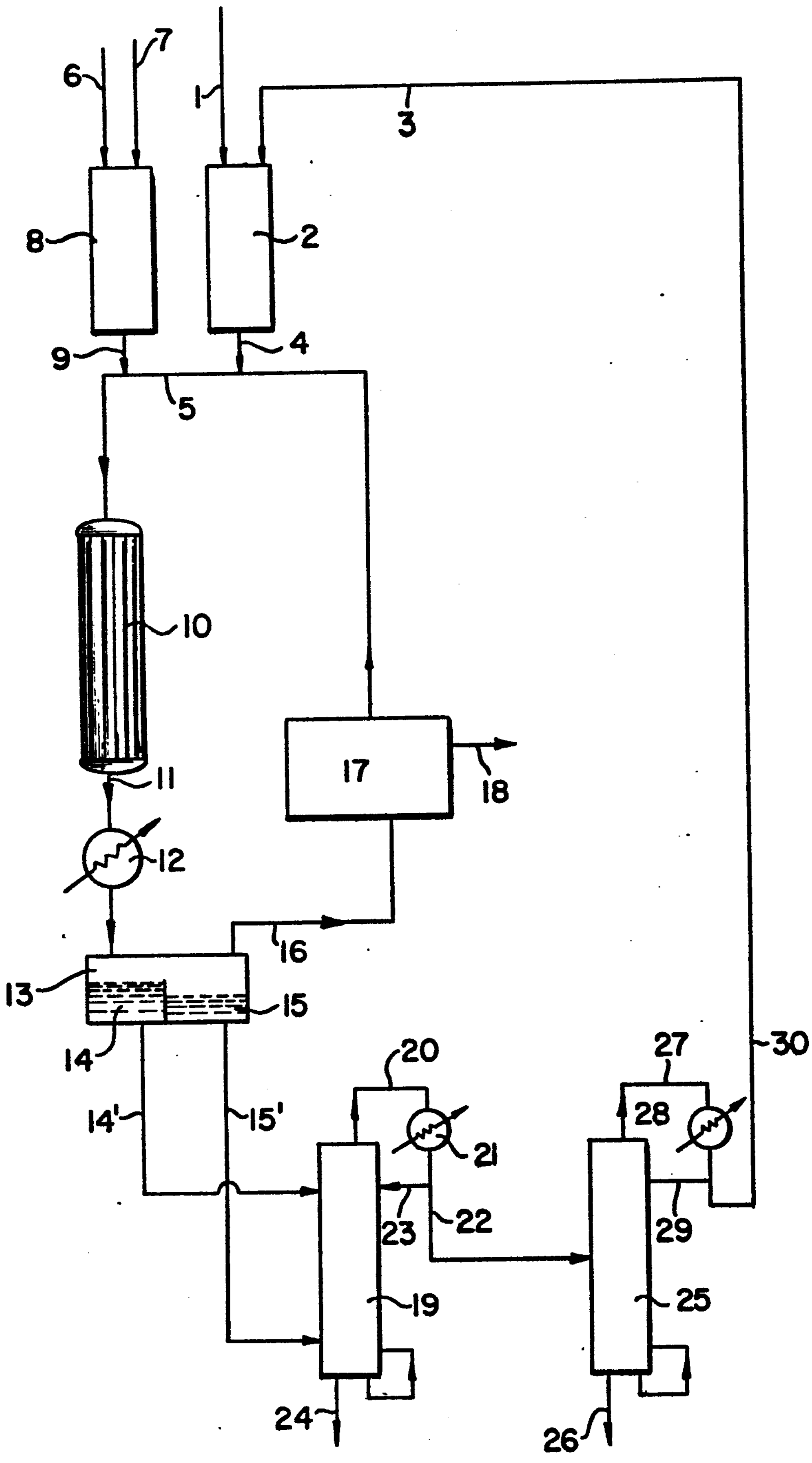

1
2
3
4
5
6
7
8
9
10
11
12
13
14
15
14'
15'
16
17
18
19
20
21
22
23
24
25
26
27
28
29
30